Europäisches Patentamt

European Patent Office

Office européen des brevets

(11) Publication number: **0 323 137**
**A1**

## (12) EUROPEAN PATENT APPLICATION

(21) Application number: 88312180.8

(22) Date of filing: 22.12.88

(51) Int. Cl.4: **C07C 41/06** , **C07C 41/09** ,
**C07C 43/04**

(30) Priority: 30.12.87 US 139543

(43) Date of publication of application:
05.07.89 Bulletin 89/27

(84) Designated Contracting States:
BE DE FR GB IT NL SE

(71) Applicant: **MOBIL OIL CORPORATION**
**150 East 42nd Street**
**New York New York 10017(US)**

(72) Inventor: **Huang, Tracy Jau-Hua**
**9 Woodfield Lane**
**Lawrenceville New Jersey 08648(US)**
Inventor: **LaPierre, Rene Bernard**
**43 Arrowhead Court**
**Medford New Jersey 08055(US)**
Inventor: **Tabak, Samuel Allen**
**204 East Pine Street**
**Wenonah New Jersey 08090(US)**

(74) Representative: **Colmer, Stephen Gary**
**Patent Department c/o Mobil Services**
**Company Limited Mobil Court 3 Clements**
**Inn**
**London WC2A 2EB(GB)**

(54) Process for the production of ethers.

(57) In a process for producing an ether, at least one light olefin is contacted with water in an olefin conversion unit in the presence of an acidic zeolite catalyst to provide an aqueous mixture of an alcohol and an ether, the olefin conversion unit being operated under conditions effective to provide the alcohol by the reaction of the olefin and water and the ether by the dehydration of the alcohol and/or by the reaction of the olefin and the alcohol. The aqueous mixture of alcohol and ether is then introduced into a distillation unit which is operated under conditions effective to provide an azeotropic overheads fraction comprising the ether, water and a minor amount of the alcohol, and a bottoms fraction comprising a major amount of the alcohol and a minor amount of the ether. Thereafter the azeotropic overheads fraction is introduced into a decanter unit which is operated under conditions which are effective to provide an ether layer containing little or no water and an aqueous product containing said alcohol, at least part of said ether layer being recycled to the distillation unit.

## PROCESS FOR THE PRODUCTION OF ETHERS

This invention relates to a process for the production of ether(s) and, optionally, mixtures of alcohol(s) and ether(s). More particularly, the invention relates to a process for the conversion of a light olefin such as ethylene, propylene, butenes, pentenes, hexenes, heptenes, and their mixtures, in a conversion unit employing an acidic zeolite as olefin conversion catalyst to produce a mixture of alcohol(s) and ether(s) and thereafter recovering the ether(s) containing at most only small amounts of water. If desired, the ether(s) can be recombined with co-produced alcohol(s) to provide substantially dry alcohol/ether mixtures in virtually any desired ratio.

There is a need for an efficient catalytic process for manufacturing ethers and alcohols from lift olefins thereby augmenting the supply of high octane blending stocks for gasoline. Lower molecular weight ethers such as diisopropyl ether (DIPE) and alcohols such as isopropyl alcohol (IPA) are in the gasoline boiling range and are known to have a high blending octane number. In addition, by-product propylene from which DIPE and IPA can be made is usually available in a fuels·refinery. The petrochemicals industry also produces mixtures of light olefin streams in the $C_2$ to $C_7$ molecular weight range and the conversion of such streams or fractions thereof to ethers and alcohols can also provide products which are useful as solvents and as blending stocks for gasoline.

The catalytic hydration of olefins to provide alcohols and ethers is a well-established art and is of significant commercial importance. Representative olefin hydration processes are disclosed in U.S. Patent Nos. 2,162,913; 2,477,380; 2,797,247; 3,798,097; 2,805,260; 2,830,090; 2,861,045; 2,891,999; 3,006,970; 3,198,752; 3,810,849 and 3,989,762.

Olefin hydration employing zeolite catalysts is also known. As disclosed in U.S. Patent No. 4,214,107, lower olefins, in particular,propylene, can be catalytically hydrated over a crystalline aluminosilicate zeolite catalyst having a silica to alumina ratio of at least 12 and a Constraint Index of 1 to 12, e.g., HZSM-5, to provide the corresponding alcohol, essentially free of ether and hydrocarbon by-product.

According to U.S. Patent No. 4,499,313, an olefin is hydrated to the corresponding alcohol in the presence of hydrogen-type mordenite or hydrogen-type zeolite Y, each having a silica-alumina molar ratio of 20 to 500. The use of such a catalyst is said to result in higher yields of alcohol than olefin hydration processes which employ conventional solid acid catalysts. Use of the zeolite catalyst is also said to offer the advantage over ion-exchange type olefin hydration catalysts of not being restricted by the hydration temperature. Reaction conditions employed in the process include a temperature of from 50-300°C, preferably 100-250°C, a pressure of 5 to 200 $kg/cm^2$ to maintain liquid phase or gas-liquid multi-phase conditions and a mole ratio of water to olefin of from 1 to 20. The reaction time can be 20 minutes to 20 hours when operating batchwise and the liquid hourly space velocity (LHSV) is usually 0.1 to 10 in the case of continuous operation.

The reaction of light olefins with alcohols to provide ethers is also well known. According to U.S. Patent No. 4,042,633, DIPE is prepared from isopropyl alcohol (IPA) employing montmorillonite clay catalysts, optionally in the presence of added propylene. U.S. Patent No. 4,175,210 discloses the use of silicatungstic acid as catalyst for the reaction of olefin(s) with alcohol to provide ether(s). As disclosed in U.S. Patent No. 4,182,914, DIPE is produced from IPA and propylene in a series of operations employing a strongly acidic cation exchange resin as catalyst. In the process for producing a gasoline blending stock described in U.S. Patent No. 4,334,890, a mixed $C_4$ stream containing isobutylene is reacted with aqueous ethanol to form a mixture of ethyl tertiary butyl ether and tertiary butanol. U.S. Patent No. 4,418,219 describes the preparation of methyl tertiary-butyl ether (MTBE), a high octane blending agent for motor fuels, by reacting isobutylene and methanol in the presence of, as catalyst, boron phosphate, blue tungsten oxide or a crystalline aluminosilicate zeolite having a silica to alumina mole ratio of at least 12:1 and a Constraint Index of 1 to 12. U.S. Patent No. 4,605,787 discloses the preparation of alkyl tert-alkyl ethers such as MTBE and methyl tert-amyl ether (MTAE) by the reaction of a primary alcohol with an olefin having a double bond on a tertiary carbon atom employing as catalyst an acidic zeolite having a Constraint Index of 1 to 12, e.g., zeolite ZSM-5, 11, 12, and 23, dealuminized zeolite Y and rare earth-exchanged zeolite Y. European Published Patent Application No. 55,045 describes a process for reacting an olefin and an alcohol to provide an ether, e.g., isobutene and methanol to provide MTBE, in the presence of an acidic zeolite catalyst such as zeolite Beta, and zeolites ZSM-5, 8, 11, 12, 23, 35, 43 and 48.

It is an object of the present invention to provide a process for converting low cost, readily available sources of light olefins to ether(s) and, optionally, mixtures of alcohol(s) and ether(s), which can be used as high octane blending stocks for gasoline.

Accordingly, the invention resides in a process for producing an ether, comprising the steps of:

a) contacting at least one light olefin with water in an olefin conversion unit in the presence of an acidic zeolite catalyst to provide an aqueous mixture of an alcohol and an ether, the olefin conversion unit being operated under conditions effective to provide the alcohol by the reaction of the olefin and water and the ether by dehydration of the alcohol and/or by reaction of the olefin and the alcohol;

b) introducing the aqueous mixture of alcohol and ether into a distillation unit, which is operated under conditions effective to provide an azeotropic overheads fraction comprising the ether, water and a minor amount of the alcohol, and a bottoms fraction comprising a major amount of the alcohol and a minor amount of the ether;

c) introducing the azeotropic overheads fraction into a decanter unit operated under conditions effective to provide an ether layer containing at most negligible amounts of water and an aqueous layer containing negligible amounts of alcohol; and,

d) recycling at least part of said ether layer to the distillation unit.

The present process offers great flexibility in providing essentially pure ethers or ethers combined with controlled quantities of alcohols.

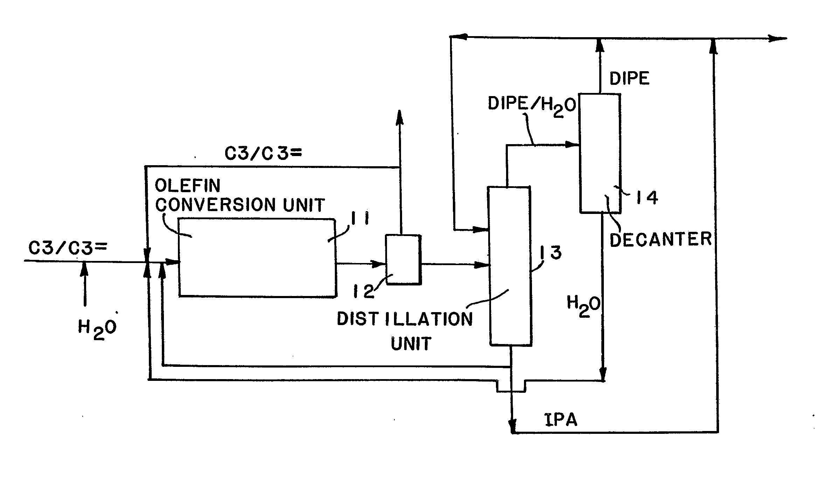

The accompanying drawing is a schematic representation of the process according to one example of the invention as applied to the production of an IPA/DIPE mixture of predetermined composition containing less than 1 weight percent water.

Referring to the drawing, the present invention is applicable to the conversion of individual light olefins and mixtures of olefins of various structure, preferably within the $C_{2-7}$ range, to ethers. Accordingly, the invention is applicable to the conversion of ethylene, propylene, butenes, pentenes, hexenes and heptenes, mixtures of these and other olefins such as gas plant off-gas containing ethylene and propylene, naphtha cracker off-gas containing light olefins, fluidized catalytic cracked (FCC) light gasoline containing pentenes, hexenes and heptenes, and refinery FCC propane/propylene streams. For example, a typical FCC light olefin stream possesses the following composition:

| Typical Refinery FCC Light Olefin Composition | | |
|---|---|---|
| | Wt.% | Mole% |
| Ethane | 3.3 | 5.1 |
| Ethylene | 0.7 | 1.2 |
| Propane | 14.5 | 15.3 |
| Propylene | 42.5 | 46.8 |
| Isobutane | 12.9 | 10.3 |
| n-Butane | 3.3 | 2.6 |
| Butenes | 22.1 | 18.3 |
| Pentanes | 0.7 | 0.4 |

The process of the invention is especially applicable to the conversion of propylene and propylene-containing streams to DIPE and IPA/DIPE mixtures containing little if any water.

The conversion of the light olefin takes place in an olefin conversion unit 11 wherein several reactions occur simultaneously to provide a mixture of alcohol and ether. Thus, olefin will react with water fed to the unit 11 to produce alcohol, alcohol will react with olefin to produce ether and/or alcohol will undergo dehydration to produce ether.

The foregoing olefin conversion reactions can be carried out under liquid phase, vapor phase or mixed vapor-liquid phase conditions in batch or in a continuous manner under stirred tank reactor or fixed bed flow reactor conditions, e.g., trickle-bed, liquid-up-flow, liquid-down-flow, counter- current flow and co-current flow.

In general, the useful olefin conversion catalysts embrace two categories of zeolite, namely, the intermediate pore size variety as represented, for example, by ZSM-5, which possess a Constraint Index (as described in U.S. Patent No. 4016218) of 2 to 12 and the large pore variety as represented, for example, by zeolites Y and Beta, which possess a Constraint Index less than about 2. Both varieties of zeolite preferably possess a framework silica-to-alumina ratio of greater than 7, usually greater than 20. The zeolite will be in the acid form and preferably will possess an alpha value of at least 1, more preferably at least 10 and most preferably at least 100. It will often be advantageous to provide the zeolite as a composite bound with a catalytically active or inactive material such as alumina or silica which is stable under the olefin conversion conditions employed.

Of particular interest for use herein are the large pore acidic zeolites, e.g., zeolite Beta, X, L, Y, USY, REY, Deal Y, ZSM-3, ZSM-4, ZSM-12, ZSM-20 and ZSM-50, with zeolite beta being particularly preferred.

The olefin hydration step can be effected under essentially any practical set of hydration conditions which provides alcohol(s) and ether(s) in appreciable amounts. However, good results can generally be obtained employing a temperature ranging from ambient (20°C) up to 300°C, preferably from 50 to 220°C and more preferably from 90 to 200°C, a total system pressure of at least 500 kPa (5 atm), preferably at least 2000 kPa (20 atm) and more preferably at least 4000 kPa (40 atm), a water to total olefin mole ratio of 0.1 to 30, preferably 0.2 to 15 and most preferably 0.3 to 5, and an LHSV of 0.1 to 10. It is to be noted that low water:olefin mole ratios, namely 0.1 - 1 and preferably 0.2 - 0.8, employing zeolite Beta as the catalyst shifts olefin hydration products towards ether(s) and away from alcohol(s).

The aqueous mixture of alcohol and ether produced in the olefin conversion unit 11 containing unconverted olefin and any inert gaseous material such as saturated hydrocarbon which may have been part of the olefin feed stream, and the small quantities of oligomer which are typically present in the reaction effluent, is then passed to a separator unit 12 to provide a gaseous phase containing the unconverted olefin and a liquid phase containing alcohol, ether, water and oligomer. The gaseous phase is recycled to the olefin conversion unit with part of it being vented off as may be necessary to avoid build-up of any inert component(s) in the system. The aqueous phase made up of alcohol, ether and oligomer is then introduced into a distillation tower 13 to provide an azeotropic overheads fraction containing most of the ether, a minor part of the alcohol and most of the water and oligomer and a bottoms fraction containing a major part of the alcohol, a minor part of the ether and oligomer and essentially no water. Part or all of the bottoms fraction can be recycled to the olefin conversion unit 11 to maintain a high level of alcohol therein as this has been found to shift selectivity to ether. Alternatively, part or all of the bottoms fraction can be recombined with product ether to provide a dry alcohol/ether mixture of just about any desired composition.

The distillation unit 13 is supplied with part or all of the ether-rich upper phase recovered from a downstream decanter unit 14 in order to reduce the water content of the final ether product even further. If all of the decanter·overhead is totally recycled, the water content in the product ether will be zero. If only part of the decanter overhead is recycled, the water content in the product ether will generally be between zero and 1 wt. %.

Following condensation of the azeotrope from the distillation tower 13, the liquid product is introduced into the decanter unit 14 where phase separation takes place. The decanter overheads are recovered as essentially dry ether and the aqueous decanter bottoms containing a small amount of alcohol can, if desired, be introduced into the olefin conversion unit 11.

The following example is illustrative of the process of the invention.

## EXAMPLE 1

The conversion of propylene contained in a propylene/propane refinery stream (70 mole % propylene, 30 mole % propane) is illustrated in the process scheme shown in the appended figure of drawing. The conditions of the propylene conversion employing an extrudate of zeolite Beta (85 wt.%) bound with silica (15 wt%) are:

160°C, 12500 kPa (1800 psig), 0.5 water to propylene mole ratio and 0.5 weight hourly space velocity (WHSV) based on propylene. The results in moles/hr of feeds/products are set forth in the following Table:

4

Table

EP 0 323 137 A1

| | IPA/DIPE Via Propylene Conversion over Zeolite Beta | | | | | | | | | | | | | | |
|---|---|---|---|---|---|---|---|---|---|---|---|---|---|---|---|
| Feed/Product Stream | Moles/Hr | | | | | | | | | | | | | | |
| | 1 | 2 | 3 | 4 | 5 | 6 | 7 | 8 | 9 | 10 | 11 | 12 | 13 | 14 | Wt% |
| Propane | 155.0 | - | 157.90 | - | 312.8 | 312.8 | 155.0 | - | - | - | - | - | - | - | - |
| Propylene | 361.6 | - | 85.8 | - | 447.4 | 170.0 | 84.2 | - | - | - | - | - | - | - | - |
| DIPE | - | - | - | 0.1 | 0.1 | - | - | 92.5 | 158.1 | 22.7 | 158.0 | 88.3 | 69.3 | 92.4 | 63.1 |
| IPA | - | - | - | 1.4 | 1.4 | - | - | 86.5 | 18.7 | 77.4 | 17.4 | 9.7 | 7.7 | 35.1 | 34.2 |
| Oligomer | - | - | - | 0.0 | 0.0 | - | - | 3.7 | 6.4 | 0.9 | 6.4 | 3.6 | 2.8 | 3.7 | 2.1 |
| Water | - | 182.5 | - | 40.8 | 223.3 | - | - | 45.8 | 52.1 | 0.0 | 11.3 | 6.3 | 5.0 | 5.0 | 0.6 |

## Claims

1. A process for producing an ether comprising the steps of:

a) contacting at least one light olefin with water in an olefin conversion unit in the presence of an acidic zeolite catalyst to provide an aqueous mixture of an alcohol and an ether, the olefin conversion unit being operated under conditions effective to provide the alcohol by the reaction of the olefin and water and the ether by the dehydration of the alcohol and/or by the reaction of the olefin and the alcohol;

b) introducing the aqueous mixture of alcohol and ether into a distillation unit which is operated under conditions effective to provide an azeotropic overheads fraction comprising the ether, water and a minor amount of the alcohol, and a bottoms fraction comprising a major amount of the alcohol and a minor amount of the ether;

c) introducing the azeotropic overheads fraction into a decanter unit which is operated under conditions which are effective to provide an ether layer containing little or no water and an aqueous product containing said alcohol; and,

d) recycling at least part of said ether layer to the distillation unit.

2. The process of Claim 1 wherein the zeolite has a Constraint Index of greater than 2.

3. The process of Claim 1 or Claim 2 wherein the zeolite is selected from zeolite Beta, X, L, Y, REY, Deal Y, ZSM-3, ZSM-4, ZSM-12, ZSM-20 and ZSM-50.

4. The process of any preceding Claim wherein the light olefin is at least one member of the group consisting of ethylene, propylene, butenes, pentenes, hexenes and heptenes.

5. The process of any preceding Claim wherein the olefin conversion unit is maintained under conditions comprising a temperature of from ambient to 300° C, an overall system pressure of at least 500 kPa, a water to total olefin mole ratio of 0.1 to 30 and an LHSV of 0.1 to 10.

6. The process of any preceding Claim wherein the zeolite is zeolite Beta and the water to total olefin mole ratio is less than about 1.

7. The process of any preceding Claim wherein the effluent from the olefin conversion unit containing unconverted olefin is introduced into a separator unit operated under conditions which are effective to provide a gaseous fraction containing unconverted olefin and a liquid fraction containing water, alcohol and ether, the unconverted olefin being recycled to the olefin conversion unit and the liquid fraction being introduced into the distillation unit.

8. The process of any preceding Claim wherein at least part of the bottoms fraction from the distillation unit is recycled to the olefin conversion unit.

9. The process of any preceding Claim wherein at least part of the bottoms fraction from the distillation unit is combined with the product ether to provide an alcohol/ether mixture.

10. The process of any preceding Claim wherein the olefin is propylene or propylene in admixture with propane and the ether product is diisopropyl ether containing less than 1 weight percent of water.

C3/C3=

H₂O

OLEFIN CONVERSION UNIT

C3/C3=

11

12

DISTILLATION UNIT

13

IPA

H₂O

DIPE/H₂O

DECANTER

14

DIPE

## DOCUMENTS CONSIDERED TO BE RELEVANT

EP 88312180.8

| Category | Citation of document with indication, where appropriate, of relevant passages | Relevant to claim | CLASSIFICATION OF THE APPLICATION (Int. Cl.4) |
|---|---|---|---|
| A | EP - A2 - 0 213 884 (MOBIL OIL) <br> * Abstract * <br> -- | 1,3,4 | C 07 C 41/06 <br> C 07 C 41/09 <br> C 07 C 43/04 |
| A | EP - A1 - 0 210 793 (THE BRITISH PETROLEUM) <br> * Claims 1,4-6 * <br> -- | 1,4 | |
| D,A | US - A - 4 605 787 (POCHEN CHU et al.) <br> * Abstract * <br> -- | 1 | |
| A | EP - A1 - 0 130 697 (TOA NENRYO KOGYO) <br> * Abstract * | 1 | |
| D,A | & US-A-4 499 313 <br> -- | | |

| | | | TECHNICAL FIELDS SEARCHED (Int. Cl.4) |
|---|---|---|---|
| A | EP - A1 - 0 063 815 (VEBA OIL AG) <br> * Claim 1 * <br> -- | 1 | C 07 C 27/00 <br> C 07 C 29/00 |
| D,A | US - A - 4 334 890 (NAND K. KOCHAR et al.) <br> * Abstract * <br> -- | 1 | C 07 C 31/00 <br> C 07 C 41/00 <br> C 07 C 43/00 |
| D,A | US - A - 4 182 914 (MASAO IMAIZUMI) <br> * Abstract * <br> -- | 1 | |
| D,A | US - A - 3 989 762 (WILHELM ESTER) <br> * Abstract * <br> ---- | 1 | |

The present search report has been drawn up for all claims

| Place of search | Date of completion of the search | Examiner |
|---|---|---|
| VIENNA | 14-03-1989 | REIF |